# EUROPEAN PATENT SPECIFICATION

(11) **EP 0 682 935 B1**
(45) Date of publication and mention of the grant of the patent: **05.03.2003**
(21) Application number: 95107435.0
(22) Date of filing: 16.05.1995
(51) Int. Cl.: A61K 7/06

(54) **Hair cosmetics**
Haarkosmetika
Cosmétiques pour les cheveux

(30) Priority: 17.05.1994 JP 10305794
(43) Date of publication of application: 22.11.1995
(73) Proprietor: KAO CORPORATION, Chuo-ku, Tokyo (JP)
(72) Inventor: Horinishi, Nobutaka, c/o Kao Corp., Res. Lab., Tokyo (JP); Yahagi, Kazuyuki, c/o Kao Corp., Res. Lab., Tokyo (JP)
(74) Representative: Hansen, Bernd, Dr. Dipl.-Chem.

(56) References cited:
- EP-A- 0 512 270
- WO-A-93/18737
- US-A- 5 180 584
- DATABASE WPI Section Ch, Week 8348 Derwent Publications Ltd., London, GB; Class D21, AN 83-829925 XP002056678 & JP 58 180 421 A (SHISEIDO CO LTD)

## Description

### FIELD OF THE INVENTION

This invention relates to hair cosmetics, more particularly to hair cosmetics which can give smooth finishing with no stickiness independent of the hair quality, and which can prevent entanglement of hair at the time of shampooing.

### BACKGROUND OF THE INVENTION

Since hair cosmetics must have a capacity to provide hair with flexibility and smoothness, quaternary ammonium salts having two long chain alkyl groups per molecule are used because of such a demand. Examples of such quaternary ammonium salts include distearyldimethylammonium chloride, di(hydrogenated tallow alkyl)dimethylammonium salt and the like. However, the prior art hair cosmetics which contain these quaternary ammonium salts do not always satisfy flexibility, smoothness and oily feeling at the time of shampooing and drying. In addition, these hair cosmetics pose a problem of causing entanglement of hair at the time of shampooing, especially in the case of thin hair.

### SUMMARY OF THE INVENTION

In view of the above, it therefore becomes an object of the present invention to resolve the aforementioned problems involved in the prior art, thereby providing a hair cosmetic which can give smooth finishing with no stickiness independent of the hair qualities such as thickness/thinness of hair and the like, and which can prevent entanglement of hair at the time of shampooing.

With the aim of achieving such an object, the inventors of the present invention have conducted intensive studies and found that the use of a specified quaternary ammonium salt is effective in producing hair cosmetics which can give smooth finishing with no stickiness even in the case of thin hair and also can prevent entanglement of hair at the time of shampooing. The present invention has been accomplished on the basis of this finding.

Accordingly, the present invention provides a hair cosmetic which contains a quaternary ammonium salt represented by the following formula (1): 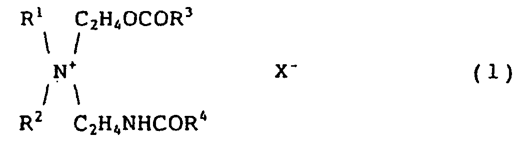 wherein R¹ and R² may be the same or different from each other and each represents an alkyl or hydroxyalkyl group having 1 to 4 carbon atoms, R³ and R⁴ may be the same or different from each other and each represents a straight-chain or branched alkyl or alkenyl group having 7 to 35 carbon atoms and X⁻ represent an anion.

The present invention also provides a hair cosmetic which contains, in addition to the above quaternary ammonium salt, at least one component selected from the group consisting of fats and oils, silicones, solvents, α-hydroxymonocarboxylic acids and liquid crystal forming base materials.

Other objects and advantages of the present invention will be made apparent as the description progresses.

### DETAILED DESCRIPTION OF THE INVENTION

The quaternary ammonium salt to be used in the present invention is a compound represented by the aforementioned formula (1).

Examples of the alkyl and hydroxyalkyl groups having 1 to 4 carbon atoms, represented by R¹ and R² in formula (1), include methyl, ethyl, n-propyl, isopropyl, butyl and the like groups and their derivatives to which one or more hydroxyl groups substitute to, such as hydroxymethyl, hydroxyethyl, hydroxypropyl and the like groups, of which methyl, ethyl and hydroxyethyl groups, especially methyl and hydroxyethyl groups, are preferred.

Examples of the straight-chain or branched alkyl and alkenyl groups represented by R³ and R⁴ having 6 to 35 carbon atoms include: alkyl groups such as hexyl, heptyl, octyl, nonyl, decyl, dodecyl, tridecyl, tetradecyl, pentadecyl, hexadecyl, heptadecyl, octadecyl, nonadecyl, eicosyl, heneicosyl, docosyl, tricosyl, tetracosyl, triaconsyl, pentatriaconsyl and the like groups and their corresponding branched alkyl groups; and alkenyl groups such as undecenyl, tridecenyl, tetradecenyl, pentadecenyl, hexadecenyl, heptadecenyl, octadecenyl, nonadecenyl, eicosenyl, heneicosenyl, docosenyl, tricosenyl, tetracosenyl, triaconsenyl, pentatriaconsenyl and the like groups and their corresponding branched alkenyl groups; as well as other corresponding alkyl groups derived from natural fats and oils such as coconut oil, palm oil, beef tallow, rapeseed oil, fish oil and the like. Of these, preferred groups are tetradecyl, hexadecyl, octadecyl, eicosyl, docosyl and their corresponding branched alkyl groups; and tetradecenyl, hexadecenyl, octadecenyl, eicosenyl, docosenyl and their corresponding branched alkenyl groups; particularly straight-chain or branched alkyl or alkenyl groups having 16 to 22 carbon atoms.

Examples of the anions represented by X⁻ include halogen ions such as Cl⁻, Br⁻ and the like and alkyl sulfate ions having 1 to 5 carbon atoms such as CH₃SO₄⁻, C₂H₅SO₄⁻, C₃H₇SO₄⁻ and the like, of which Cl⁻, Br⁻, CH₃SO₄⁻ and C₂H₅SO₄⁻ are preferred.

Compounds represented by the following formulae are illustrative and preferred examples of the quaternary ammonium salt represented by formula (1). 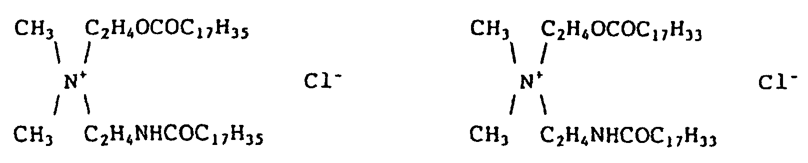 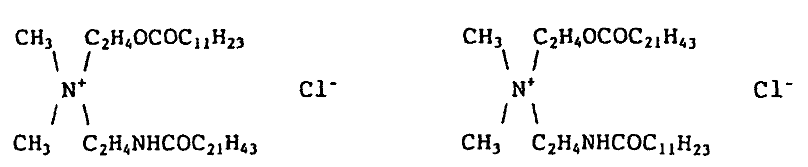 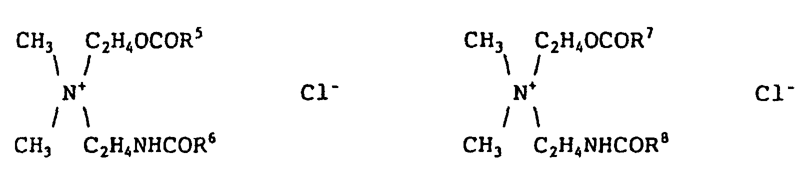 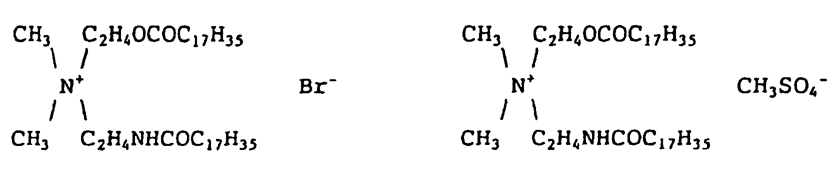 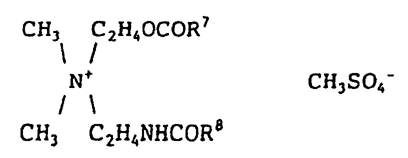 In the above formulae, R⁵ and R⁶ represent an alkyl group composition obtained by removing carboxyl group from hydrogenated tallow fatty acids and R⁷ and R⁸ represent an alkyl group composition obtained by removing carboxyl group from hydrogenated palm oil fatty acids.

The quaternary ammonium salt represented by formula (1) can be produced, for example, in accordance with the following reaction formula.

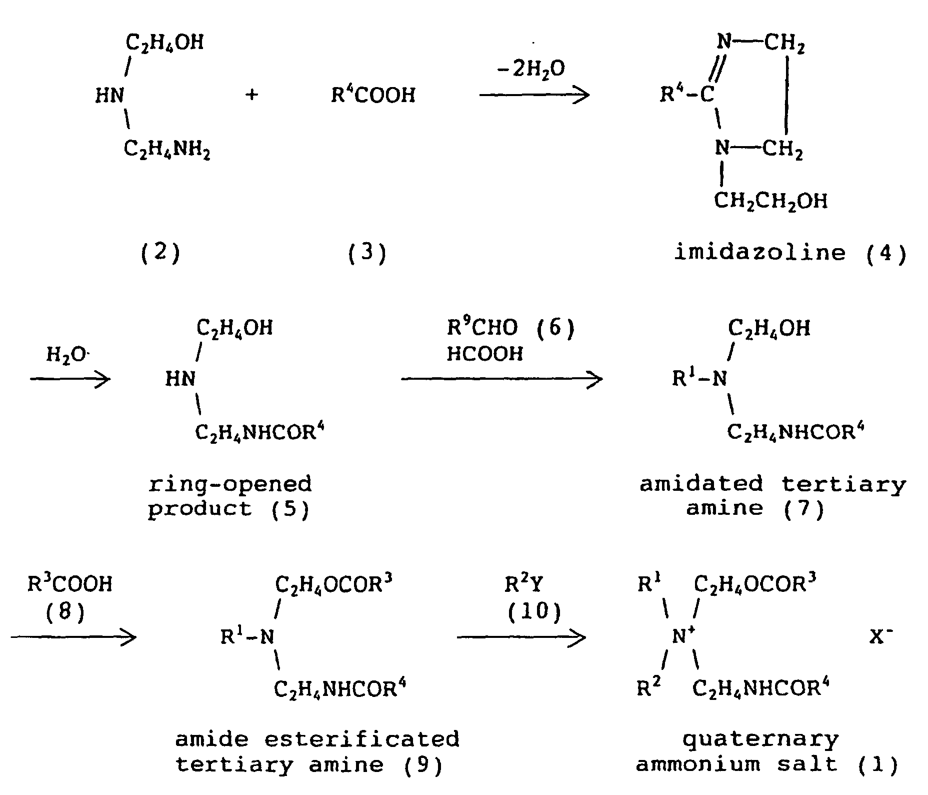

In the above formulae, R¹, R², R³, R⁴ and X⁻ are as defined in the foregoing, R⁹ represents hydrogen atom or an alkyl or hydroxyalkyl group having 1 to 3 carbon atoms, and Y represents a halogen atom or R²SO₄ group.

That is, N-(2-aminoethylethanol)amine (2) is allowed to react with a fatty acid (3) to form an imidazoline compound (4) which is subsequently hydrolyzed to form a ring-opened product (5) and then subjected to Leukart reaction using an aldehyde (6) to obtain an amidated tertiary amine (7). Next, the amine compound (7) is converted into an amide esterificated tertiary amine (9) using a fatty acid (8) and then into a quaternary ammonium salt (1).

Firstly, N-(2-aminoethylethanol)amine (2) is allowed to react with a fatty acid (3) to obtain an imidazoline compound (4). Examples of the fatty acid (3) to be used in this reaction include those which are derived from natural fats and oils such as coconut oil, palm oil, beef tallow, rapeseed oil, fish oil and the like, as well as chemically synthesized fatty acids having 7 to 36 carbon atoms. Preferably, this reaction may be carried out at an N-(2-aminoethylethanol)amine (2)/fatty acid (3) molar ratio of from 1.0 to 2.0, more preferably from 1.1 to 1.5, at a temperature of from 150 to 250°C, more preferably from 180 to 230°C. Also, in order to increase dehydration efficiency, it is desirable to reduce pressure of the reaction system gradually from atmospheric pressure to about 5 Torr.

Next, a ring-opened product (5) is obtained by hydrolyzing the imidazoline compound (4) in a water/ethanol or the like solvent mixture in the presence of sodium hydroxide or the like as a catalyst. In this case, water may be used in an amount of from 1 to 10 moles, preferably from 2 to 5 moles, per 1 mole of the imidazoline compound (4), and ethanol may be used in an amount of from 5 to 50 % by weight, preferably from 10 to 30 % by weight, based on the total weight of the imidazoline compound (4) and water. Sodium hydroxide may be used in an amount of from 0.5 to 10 mol %, preferably from 1 to 5 mol %, based on the imidazoline compound (4).

The thus obtained ring-opened product (5) is converted into an amidated tertiary amine (7) by subjecting the product to Leukart reaction under usual conditions using an aldehyde (6) such as formaldehyde, acetaldehyde or the like. The amidated tertiary amine (7) is then allowed to undergo esterification reaction with a fatty acid (8) to obtain an amide esterificated tertiary amine (9). Examples of the fatty acid (8) to be used in this reaction are the same as those illustrated as the fatty acid (3).

Thereafter, a quaternary ammonium salt (1) is produced by subjecting the thus obtained amide esterificated tertiary amine (9) to a quaternarization reaction in the usual way using a quaternarization agent (10), followed, if necessary, by counter ion exchange. Examples of the quaternarization agent (10) include alkyl halides such as methyl chloride, ethyl chloride, methyl bromide and the like and dialkyl sulfates such as dimethyl sulfate, diethyl sulfate and the like.

These quaternary ammonium salts may be used alone or as a mixture of two or more of them in an amount of preferably 0.1 % by weight or more, more preferably from 0.1 to 20 % by weight, furthermore preferably from 0.1 to 10 % by weight, based on the total composition.

The hair cosmetics of the present invention may be blended with other quaternary ammonium salt represented by the following formula (11): 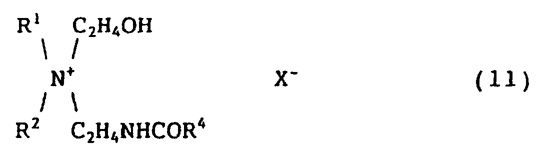 wherein R¹, R², R⁴ and X⁻ are as defined in the foregoing.

Preferred examples of the quaternary ammonium salt are those represented by the following formulae.

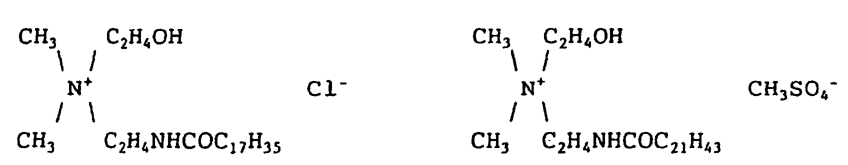

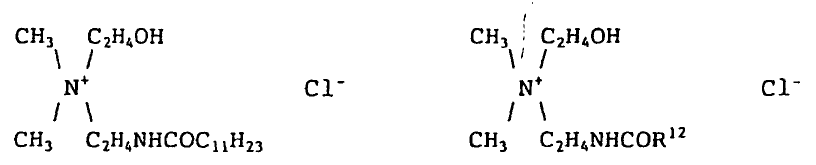

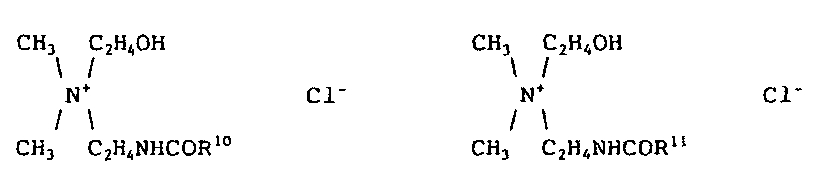

In the above formulae, R¹⁰ represents an alkyl group composition obtained by removing carboxyl group from hydrogenated tallow fatty acids, R¹¹ represents an alkyl group composition obtained by removing carboxyl group from hydrogenated palm oil and R¹² represents an alkyl group composition obtained by removing carboxyl group from oleic acid.

The hair cosmetics of the present invention may be blended further with at least one component selected from the group consisting of fats and oils , silicones, solvents, α-hydroxymonocarboxylic acids and liquid crystal forming base materials which are effective in further improving the effects of the present invention. These components may be used alone or as a mixture of two or more of them, with taking properties of each component into consideration.

Examples of fats and oils include higher alcohols having straight-chain or branched alkyl or alkenyl groups, hydrocarbons such as liquid paraffin, vaseline, solid paraffin and the like, lanolin derivatives such as liquid lanolin, lanolin fatty acid and the like, higher fatty acid esters, straight-chain or branched saturated or unsaturated higher fatty acids having 8 to 36 carbon atoms, long chain amide amine and the like fats and oils having alkyl or alkenyl groups, and animal and plant fats and oils such as minke oil, olive oil and the like. Of these fats and oils, preferred examples include monoglycerides derived from saturated or unsaturated straight-chain or branched fatty acids having 12 to 24 carbon atoms and straight-chain or branched alkyl or alkenyl group-containing higher alcohols and higher fatty acids having 12 to 26 carbon atoms. Particularly preferred examples include fatty acid monoglycerides such as oleic acid monoglyceride, palmitic acid monoglyceride, behenic acid monoglyceride, isostearic acid monoglyceride and the like; higher alcohols such as cetyl alcohol, stearyl alcohol, arachinyl alcohol, behenyl alcohol, caranarbil alcohol, ceryl alcohol and the like; and fatty acids having alkyl composition, such as octadecanoic acid, hexadecanoic acid, tetradecanoic acid, dodecanoic acid, octanoic acid, oleic acid, behenic acid, isostearic acid and 18-methyleicosanoic acid, as well as those which are derived from coconut oil, palm oil, beef tallow, rapeseed oil, fish oil and the like natural fats and oils.

These fats and oils may be blended in an amount of preferably 0.01 % by weight or more, more preferably from 0.01 to 30 % by weight, furthermore preferably from 0.05 to 20 % by weight, based on the total composition.

Examples of the silicone include the following compounds (i) to (xi):
(i) Dimethylpolysiloxane represented by the following formula (12). 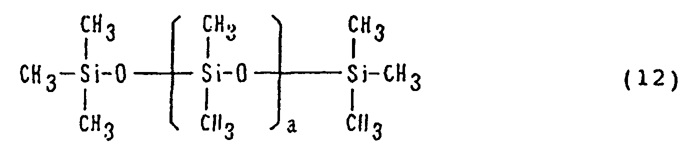
   In the above formula, a is a number of 3 to 20,000.
(ii) Methylphenylpolysiloxane represented by the following formula (13) or (14). 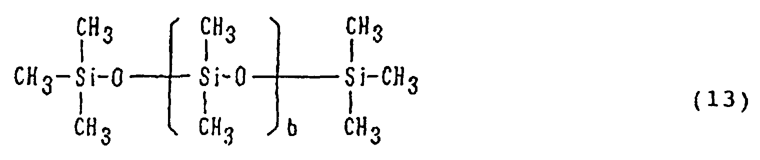 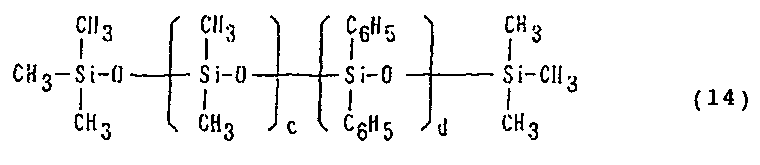
   In the above formulae, b is a number of 1 to 20,000, and c and d are numbers to give the sum of which being 1 to 500.
(iii) Amino modified silicone represented by the following formula (15) or (16). 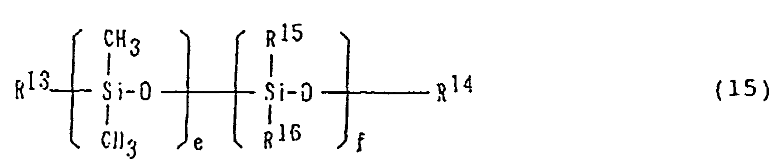 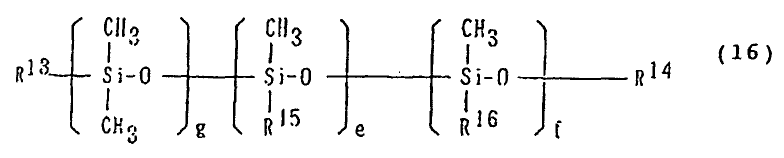
   In the above formulae, R¹³ represents methyl group or hydroxyl group, R¹⁴ represents methyl group or hydrogen atom, R¹⁵ represents an aminoalkyl group represented by 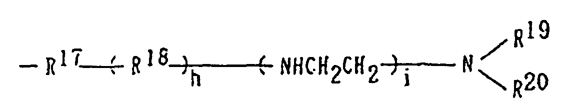 or 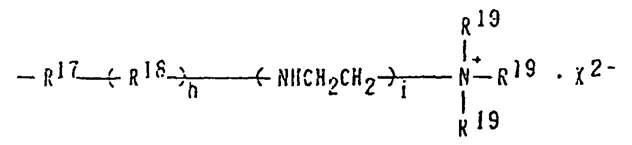 (wherein R¹⁷ represents a divalent hydrocarbon group, R¹⁸ represents a group -OCH₂CH₂-, 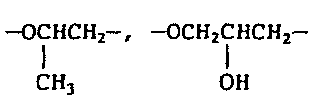 or 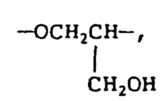 each of R¹⁹ and R²⁰ represents hydrogen atom or a monovalent hydrocarbon group, each of h and i is an integer of 0 to 6 and X²⁻ represents a halogen ion or an organic anion), R¹⁶ represents hydroxyl group, a hydroxyalkyl group, an oxyalkylene group, a polyoxyalkylene group or methyl group, and e, f and g are molecular weight-depending integers.
   Particularly preferred amino modified silicone is a compound represented by the following formula. 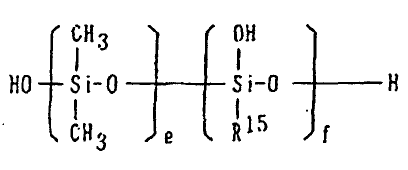
   In the above formula, R¹⁵, e and f are as defined in the foregoing.
   A specific and preferred example of the amino modified silicone to be used in the present invention is a compound represented by the following formula which has an average molecular weight of approximately from 3,000 to 100,000 and is disclosed under the name of Amodimethicone in CTFA Dictionary (Cosmetic Ingredient Dictionary, U.S.A., 3rd ed.). 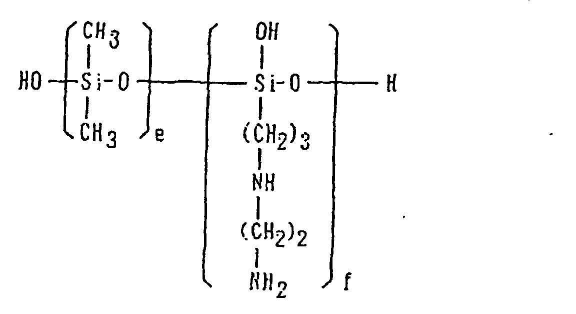
   In the above formula, e and f are as defined in the foregoing.
   Preferably, the amino modified silicone described above is used as an aqueous emulsion which can be obtained for example by the procedure disclosed in JP-B-56-38609 (the term "JP-B" as used herein means an "examined Japanese patent publication") in which a cyclic diorganopolysiloxane and an organodialkoxysilane having an aminoalkyl group and hydroxyl group, a hydroxyalkyl group, an oxyalkylene group or a polyoxyalkylene group are subjected to emulsion polymerization in the presence of a quaternary ammonium salt surfactant and water.
   When the amino modified silicone is used as an aqueous emulsion, the aqueous emulsion may contain the amino modified silicone in an amount of generally from 20 to 60 % by weight, preferably from 30 to 50 % by weight.
   Example of commercially available and preferred amino modified silicone aqueous emulsion include SM 8702C (manufactured by Toray Silicone), DC 929 (manufactured by Dow Corning) and the like.
(iv) Fatty acid modified polysiloxane represented by the following formula (17). 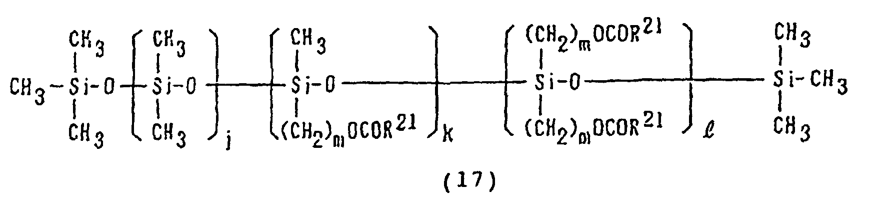
   In the above formula, each of j, k and 1 is a number of 1 to 350, m is a number of 0 to 10 and R²¹ is a group represented by CₙH₂ₙ₊₁ (n = 9 to 21).
(v) Alcohol modified silicone represented by the following formula (18) or (19). 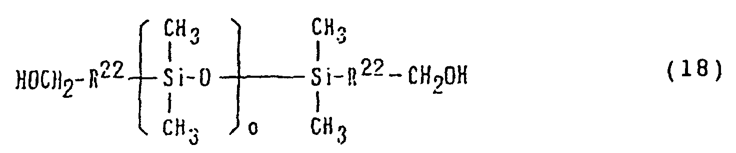 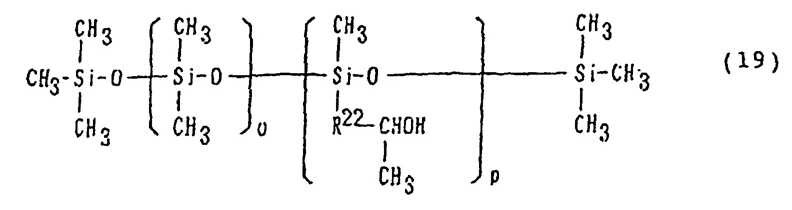
   In the above formulae, each of o and p is a number of 1 to 500 (preferably 1 to 200) and R²² is a group represented by C_{q}H_{2q} (q = 0 to 4).
(vi) Aliphatic alcohol modified polysiloxane represented by the following formula (20). 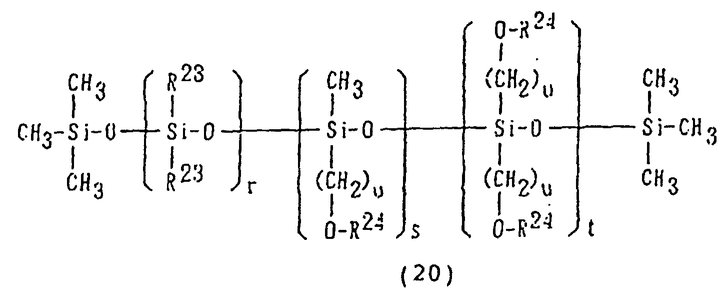
   In the above formula, R²³ represents methyl group or phenyl group, r is an integer of 1 to 3,000, s and t are integers where s + t = 1 to 500, R²⁴ represents an alkyl group having 1 to 28 (preferably 12 to 22) carbon atoms and u is an integer of 0 to 6.
(vii) Polyether modified silicone represented by the following formula (21), (22), (23), or (24). 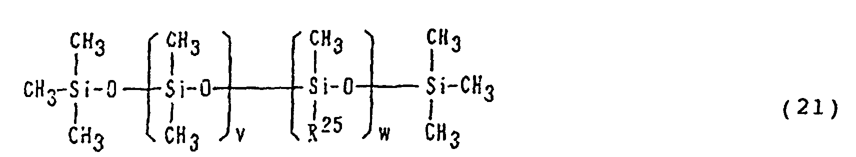 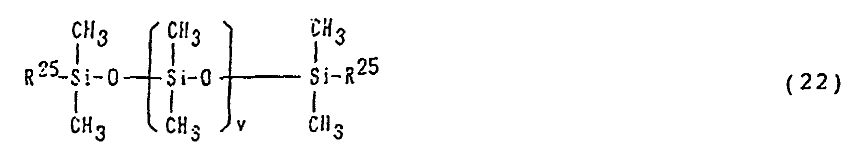 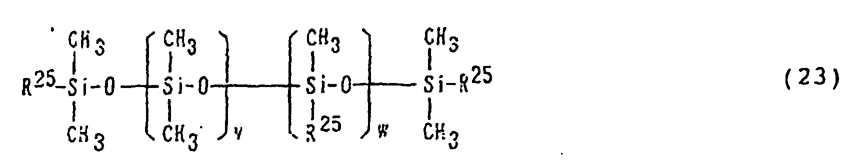 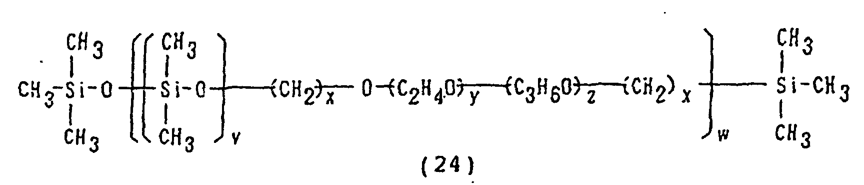
   In the above formulae, R²⁵ is a group represented by -(CH₂)ₓ-O-(C₂H₄O)_{y}-(C₃H₆O)_{z}-B (B represents an alkyl group having 1 to 12 carbon atoms or hydrogen atom), v is a number of 1 to 2,000, w is a number of 1 to 1,000, x is a number of 0 to 10, y is a number of 0 to 50 and z is a number of 0 to 50 (y + z ≥ 1).
(viii) Epoxy modified silicone represented by the following formula (25). 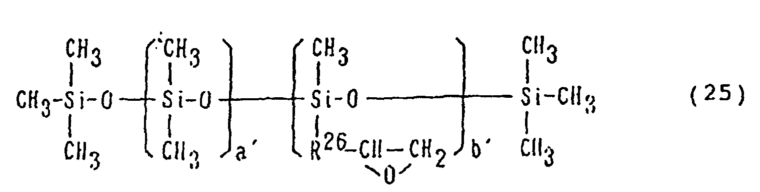
   In the above formula, a' is a number of 1 to 500 (preferably 1 to 250), b' is a number of 1 to 50 (preferably 1 to 30) and R²⁶ represents an alkylene group having 1 to 3 carbon atoms.
(ix) Fluorine modified silicone represented by the following formula (26). 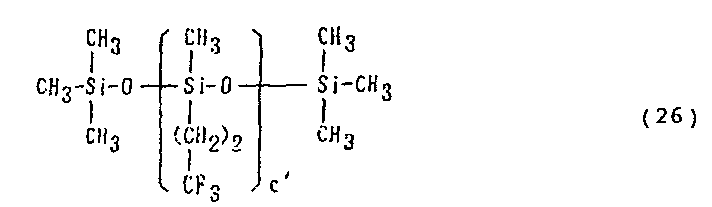
   In the above formula, c' is a number of 1 to 400 (preferably 1 to 250).
(x) Cyclic silicone represented by the following formula (27). 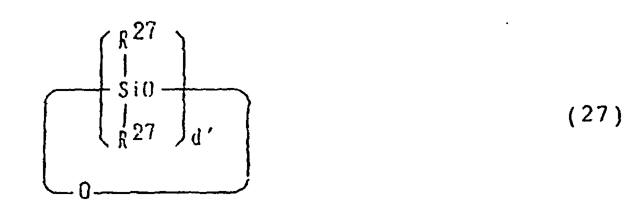
   In the above formula, d' is a number of 3 to 8 and R²⁷ represents an alkyl group having 1 to 3 carbon atoms.
(xi) Alkyl modified silicone represented by the following formula (28) or (29).

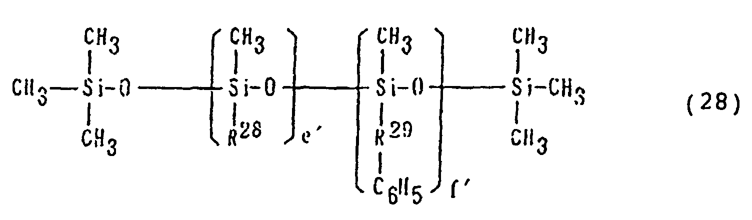

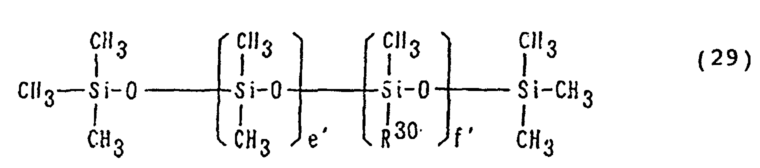

In the above formulae, each of e' and f' is a number of 1 to 500 (preferably 1 to 200), R²⁸ represents an alkyl group having 2 to 18 carbon atoms, R²⁹ is a group represented by C_{g},H_{2g}, (g' = 0 to 4) and R³⁰ represents an alkyl group having 10 to 16 carbon atoms.

Of the above silicones, those of (i), (iii), (vi), (vii) and (x) are preferred in the case of rinse-out type hair cosmetics such as rinses, conditioners and the like. In this case, "a" in the formula (12) of the silicone (i) can be selected within the range of from 3 to 20,000 depending on the intended finish feeling, but a number of approximately 100 to 1,000 is preferred for a light finishing type. In the case of non-rinse type hair cosmetics such as hair creams, styling lotions, styling mousses and the like, the silicones of (i), (ii), (iii), (vii) and (x) are preferred. In this case, "a" in the formula (12) of the silicone (i) is preferably a number of 2,000 to 8,000 for the purpose of reducing oily feeling.

When these silicones are used, they may be blended in an amount of preferably from 0.01 to 20 % by weight, more preferably from 0.1 to 10 % by weight, based on the total composition.

Examples of the solvent to be used in the present invention include compounds represented by the following formula (30).

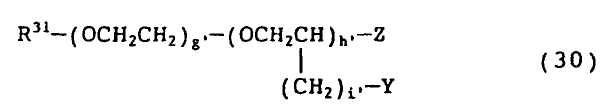

In the above formula, R³¹ represents hydrogen atom, a lower alkyl group or a group represented by 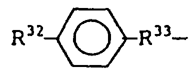 (R³² represents hydrogen atom, methyl group or methoxy group and R³³ represents a bond or a saturated or unsaturated divalent hydrocarbon radical having 1 to 3 carbon atoms), each of Y and Z represents hydrogen atom or hydroxyl group and each of g', h' and i' is an integer of 0 to 5, provided that a case in which g'=h'=i'=0 and Z=H, and a case in which g'=h'=i'=0, R³²=H and Z=OH are excluded.

Specific examples of such solvents include ethanol, isopropanol, n-propanol, n-butanol, isobutanol, ethylene glycol, propylene glycol, 1,3-butanediol, benzyl alcohol, cinnamyl alcohol, phenetyl alcohol, p-anisyl alcohol, p-methylbenzyl alcohol, phenoxyethyl alcohol, 2-benzyloxyethyl alcohol, methylcarbitol, ethylcarbitol, propylcarbitol, butylcarbitol, glycerol, N-methylpyrrolidone, N-octylpyrrolidone, N-laurylpyrrolidone, diethylene glycol monomethyl ether, diethylene glycol monoethyl ether, diethylene glycol monopropyl ether, diethylene glycol monobutyl ether, diethylene glycol monopentyl ether, diethylene glycol monoisopropyl ether, diethylene glycol mono-t-butyl ether, dipropylene glycol monomethyl ether, dipropylene glycol monoethyl ether, dipropylene glycol monopropyl ether, dipropylene glycol monobutyl ether, dipropylene glycol monopentyl ether, dipropylene glycol monoisopropyl ether, dipropylene glycol mono-t-butyl ether, triethylene glycol monoethyl ether, triethylene glycol monobutyl ether and the like, of which diethylene glycol monoethyl ether, diethylene glycol monopropyl ether, diethylene glycol monobutyl ether, benzyl alcohol, 2-benzyloxyethyl alcohol, ethanol, isopropanol, glycerol and propylene glycol are preferred.

When these solvents are used, they may be blended in an amount of preferably 0.1 % by weight or more, more preferably from 0.1 to 90 % by weight, furthermore preferably from 0.5 to 50 % by weight, based on the total composition.

Compounds represented by the following formula (31) may be used as the α-hydroxymonocarboxylic acids and their salts.

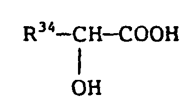

In the above formula, R³⁴ represents hydrogen atom or an alkyl group having 1 to 3 carbon atoms.

Specific examples thereof include glycolic acid, lactic acid, α-hydroxybutylic acid and the like. Their salts include sodium, potassium, calcium, ammonium, triethanolamine, organic quaternary ammonium and the like salts. Of these, glycolic acid and lactic acid and their sodium salts, potassium salts and triethanolamine salts are particularly preferred because of their excellent effects in providing hair with flexibility and preventing hair from causing entanglement at the time of washing.

When the α-hydroxymonocarboxylic acids are used, they may be blended in an amount of preferably 0.1 % by weight or more, more preferably from 0.1 to 30 % by weight, furthermore preferably from 0.1 to 20 % by weight, based on the total composition.

Examples of the liquid crystal forming base material include those which have at least one long chain branched alkyl or alkenyl group and at least three hydroxyl groups per molecule, preferably capable of keeping a lamella crystal structure at a temperature of 25°C and more than 50°C. Illustratively, (a) glycerylated polyols, (b) methyl branched fatty acid esters, (c) branched fatty acid glyceroglycolipids and the like are preferred.
(a) Examples of the glycerylated polyols include those represented by the following formula:

   Aₓ(G)

   wherein G is a residue resulting from the elimination of x hydroxyl group(s) from a polyol selected from pentaerythritol, sorbitol, maltitol, glucose, fructose and alkyl glycosides, A is a group represented by 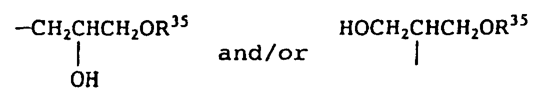 (wherein R³⁵ represents a branched alkyl or alkenyl group having 10 to 36 carbon atoms) and x is a number of at least 1 but which does not exceed the total number of hydroxyl groups of the polyol.
   Examples of the alkyl glycosides represented by G include methyl glycoside, ethyl glycoside, propyl glycoside, octyl glycoside, methyl maltoside, ethyl maltoside and the like. As R³⁵, branched alkyl groups having 16 to 36 carbon atoms, particularly 18 to 24 carbon atoms, are preferred. Examples of the branched alkyl groups are those represented by the following formula: 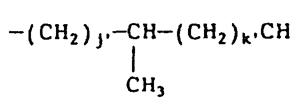 wherein each of j' and k' is an integer of 0 to 20, with the proviso that the total of j' and k' is 6 to 20; or by the following formula: 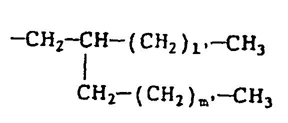 wherein each of l' and m' is an integer of 0 to 18, with the proviso that the total of l' and m' is 4 to 18.
   Specific examples thereof include methylpentadecyl, methylhexadecyl, methylheptadecyl (isostearyl), methyloctadecyl, methylbehenyl, ethylhexadecyl, ethyloctadecyl, ethylbehenyl, butyldodecyl, butylhexadecyl, butyloctadecyl, hexyldecyl, heptylundecyl, octyldodecyl, decyldodecyl, decyltetradecyl, dodecylhexadecyl, tetradecyloctadecyl and the like groups. Preferably, x is 1 or 2.
(b) Examples of the methyl branched fatty acid esters include those represented by the following formula: 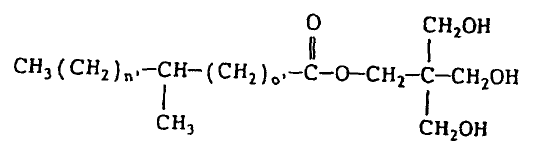 wherein each of n' and o' is an integer of 0 to 20, with the proviso that the total of n' and o' is 1 to 20. The total of n' and o' is preferably 10 to 16, more preferably 14. Also preferably, the branched methyl group may be located at a position close to the center of the alkyl main chain.
(c) Examples of the branched fatty acid glyceroglycolipids include those represented by the following formula: 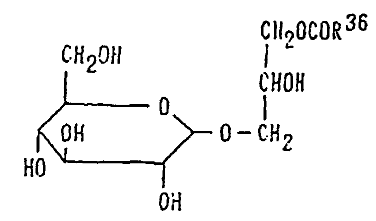 wherein R³⁶ is a group represented by 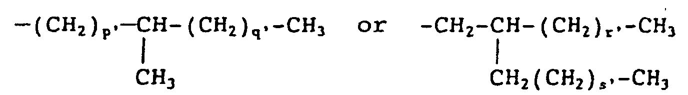 where each of p' and q' is an integer of 0 to 20 and each of r' and s' is an integer of 0 to 18, with the proviso that the total of p' and q' is 6 to 20 and that of r' and s' is 4 to 18. Preferably, the total of p' and q' is 10 to 16, more preferably 14, and the total of r' and s' is 10 to 16, more preferably 14.

When these liquid crystal forming base materials are used, they may be blended in an amount of preferably 0.01 % by weight or more, more preferably from 0.1 to 60 % by weight, furthermore preferably from 0.1 to 30 % by weight, based on the total composition.

In addition, when fats and oils, silicones, solvents, α-hydroxymonocarboxylic acids and liquid crystal forming base materials are used as a mixture of two or more of them, they may be used in a total amount of preferably from 0.05 to 70 % by weight, more preferably from 0.1 to 50 % by weight, based on the total composition.

In addition to the aforementioned components, the hair cosmetics of the present invention may further comprise, as occasion demands, cationic and nonionic surfactants such as conventionally used mono or di(long chain alkyl) quaternary ammonium salts and the like, moisture keeping agents such as glycerol, urea and the like, high molecular compounds such as cationic polymers, polysaccharides, polypeptides and the like, as well as aromatic sulfonic acids, pigments, perfumes, propellants, chelating agents, pH adjusting agents, antiseptics, anti-dandruff agents and the like, within such a range that the purpose of the present invention is not spoiled.

The hair cosmetics of the present invention can be produced in the usual way. They can be made into desired preparation forms with no particular limitation, such as aqueous solutions, ethanol solutions, emulsions, suspension, gels, liquid crystals, solids, aerosols and the like.

The hair cosmetics of the present invention can be used in hair rinses, hair conditioners, hair treatments, hair packs, hair creams, styling lotions, styling mousses, conditioning mousses, hair mousses, hair sprays, shampoos, non-rinse conditioning agents, permanent or basic hair colors, permanent agents and the like.

Since the hair cosmetics of the present invention contain the quaternary ammonium salt represented by formula (1), smooth finishing can be made with no stickiness independent of the hair quality, and they are especially excellent in preventing entanglement of hair at the time of shampooing.

In addition, these effects can be further improved by blending at least one component selected from the group consisting of fats and oils, silicones, solvents, α-hydroxymonocarboxylic acids, liquid crystal forming base materials and other quaternary ammonium salts.

Examples of the present invention are given below by way of illustration and not by way of limitation.

### SYNTHESIS EXAMPLE 1

### (Synthesis of quaternary ammonium salt (compound (1))

A four neck flask equipped with a stirrer, a thermometer and a condenser tube was charged with 284 g of stearic acid and 134 g of N-(2-aminoethylethanol)amine and heated to 200°C. Thereafter, the contents were allowed to react for 8 hours under a reduced pressure of 600 Torr and then stirred for 2 hours under a reduced pressure of 5 Torr. After cooling, to this were added 150 ml of ethanol, 54 g of water and 1.2 g of sodium hydroxide. After 2 hours of reaction at 80°C, the reaction product was recrystallized from ethanol to obtain 200 g of an amide amine represented by the following formula.

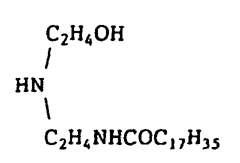

Next, a four neck flask equipped with a stirrer, a thermometer and a condenser tube was charged with 111 g of the amide amine obtained above and 150 ml of isopropyl alcohol and heated to 80°C. At the same temperature, to this were added dropwise 28 g of 35% formalin and 15.5 g of formic acid in that order. After 4 hours of reaction at 80°C, the reaction mixture was washed three times with 100 ml of water. By recrystallizing from acetone, 80 g of a methylated product represented by the following formula was obtained.

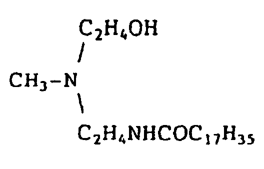

Next, a four neck flask equipped with a stirrer, a thermometer and a condenser tube was charged with 77 g of the methylated product obtained above and 60 g of stearic acid, and 10 hours of reaction was carried out at 180°C to obtain a compound represented by the following formula.

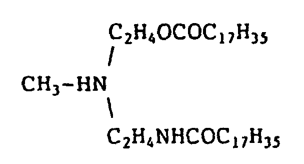

Next, 75 g of this compound and 21 g of isopropyl alcohol were put into an autoclave into which was subsequently introduced 7 g of chloromethane with a pressure. After 8 hours of reaction at 90°C, the resulting reaction product was recrystallized from acetone to obtain 70 g of a quaternary ammonium salt (1-1) represented by a formula shown in Table 1 below.

### SYNTHESIS EXAMPLES 2 TO 6

Quaternary ammonium salts (1-2) to (1-6) having respective formulae shown in Table 1 below were obtained in the same manner as described in Synthesis Example 1. In the formulae shown in Table 1, each of R³⁷ and R³⁸ represents an alkyl group composition obtained by removing carboxyl group from hydrogenated tallow fatty acids, each of R³⁹ and R⁴⁰ represents an alkyl group composition obtained by removing carboxyl group from hydrogenated palm oil fatty acids and R⁴¹ represents an alkyl group composition obtained by removing carboxyl group from oleic acid.

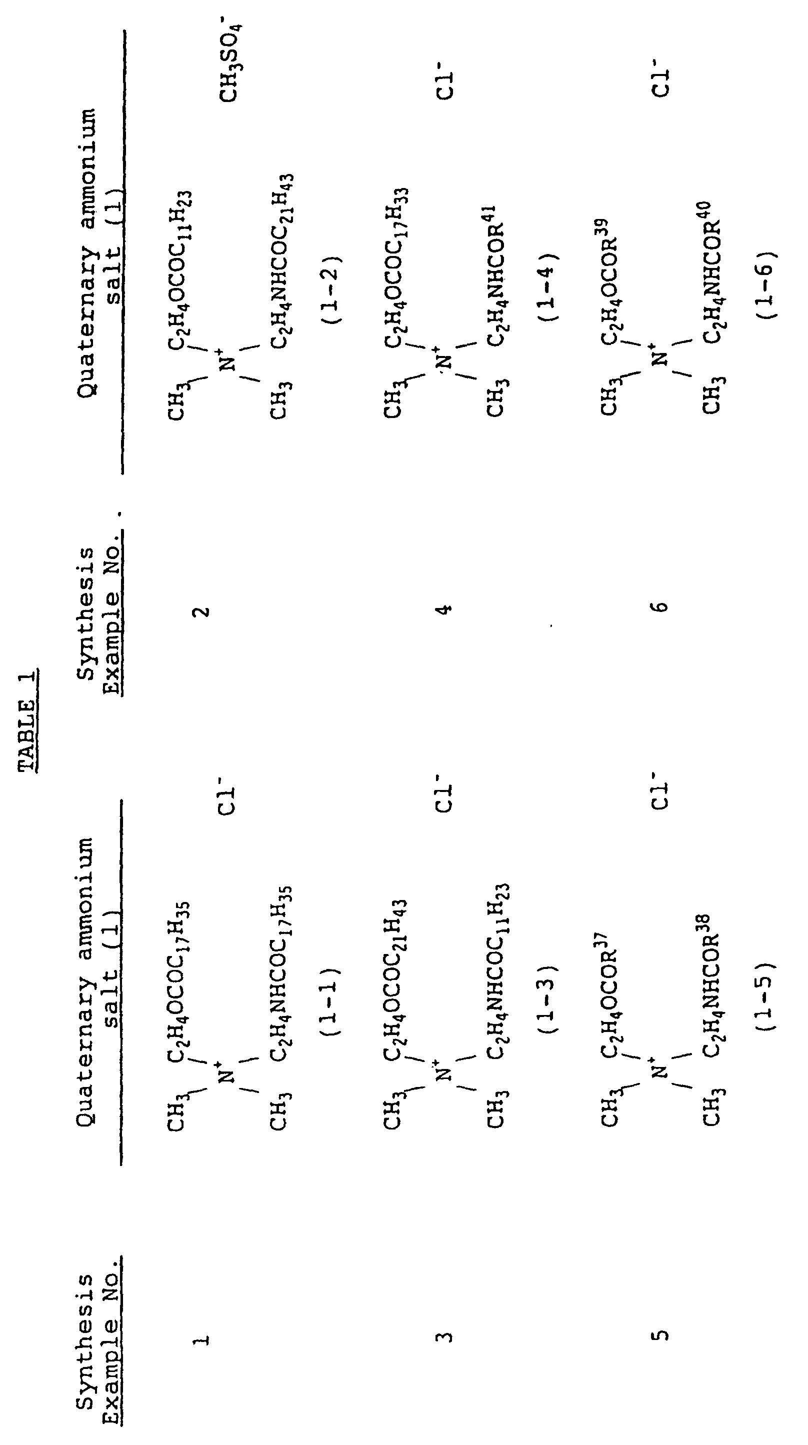

### EXAMPLE 1

Hair rinses having respective compositions shown in Table 2 (Products of the Invention 1 to 8 and Comparative Products 1 and 2) were produced in the usual way, and their smoothness and the like properties were organoleptic evaluated in accordance with the following procedure. The results are shown in Table 2.

### (Evaluation method)

A 25 g Japanese female hair (10 cm in length and 60 µm in average diameter) which has not been subjected to cold perm nor the like chemical treatments was tied up into a bundle. In the case of rinse type hair cosmetics, the hair bundle was evenly spread with 2.5 g of each cosmetic, rinsed with running water for 30 seconds and then dried using a towel and a dryer in that order. The thus dried hair was evaluated in terms of smoothness and stickiness based on the following criteria. Thereafter, the thus evaluated hair was washed with a commercially available shampoo consisting mainly of an anionic surfactant to evaluate entanglement of hair caused by the washing, based on the following criteria.

Smoothness after drying (Smoothness):
A: smooth
B: equivocal
C: not smooth

Stickiness after drying (Stickiness):
A: less sticky
B: equivocal
C: very sticky

Entanglement of hair when subsequently shampooing (Entanglement):
A: hardly entangled
B: equivocal
C: easy to be entangled

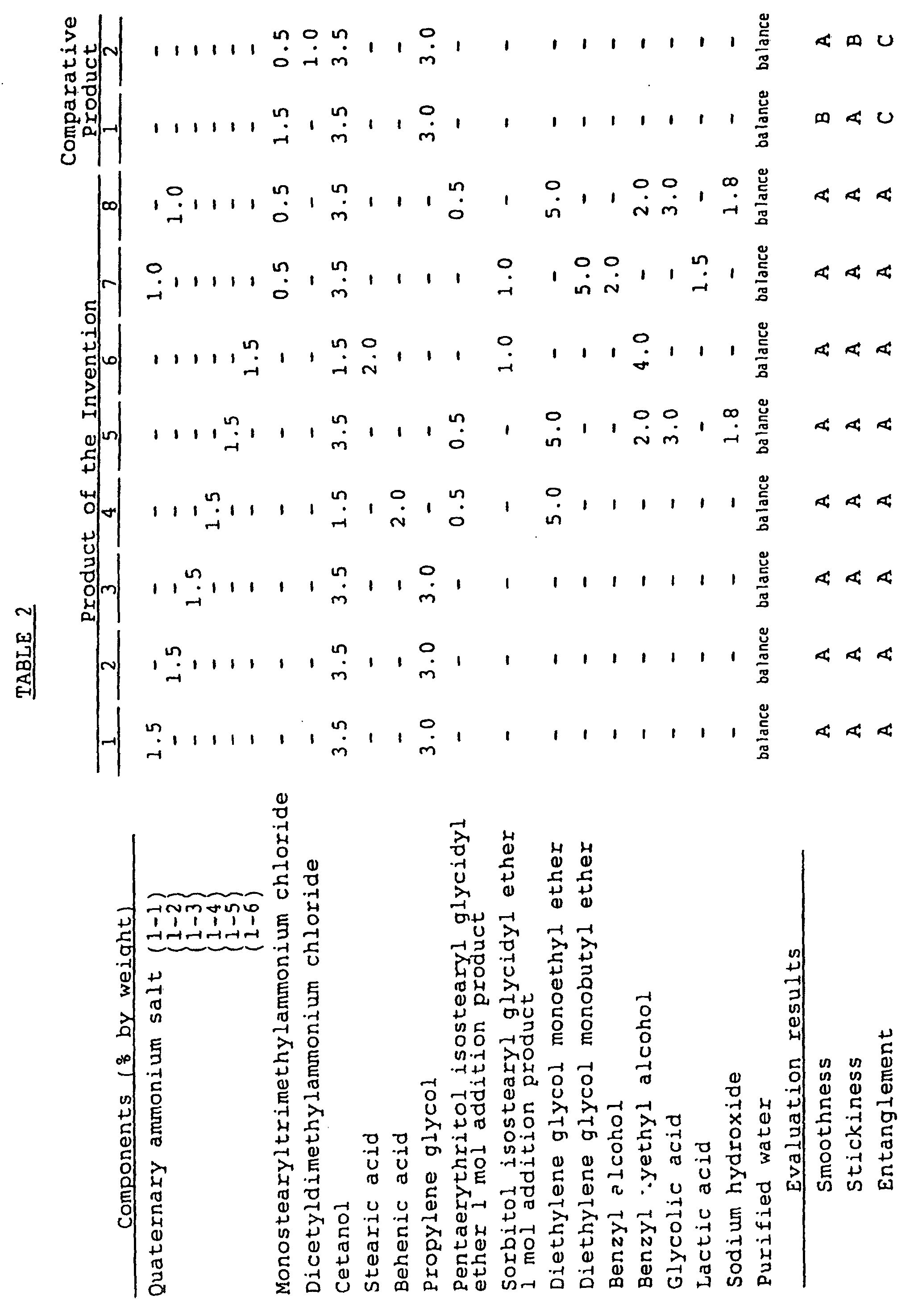

### EXAMPLE 2

Hair rinses having respective compositions shown in Table 3 (Product of the Invention 9 and Comparative Product 3) were produced in the usual way.

**TABLE 3**

| (Product of the Invention 9) | |
|---|---|
| Component | Amount (% by weight) |
| Quaternary ammonium salt (1-1) | 1.0 |
| Cetyl alcohol | 3.0 |
| Diethylene glycol monoethyl ether | 3.0 |
| Hydroxyethylcellulose | 0.5 |
| Water | balance |
| Perfume | 0.4 |
| | |

| (Comparative Product 3) | |
|---|---|
| Component | Amount (% by weight) |
| Stearyl trimethylammonium chloride | 1.0 |
| Cetyl alcohol | 3.0 |
| Diethylene glycol monoethyl ether | 3.0 |
| Hydroxyethylcellulose | 0.5 |
| Water | balance |
| Perfume | 0.4 |

Rinsing effects of these hair rinses on permed hair of each of 10 panelists were evaluated by a paired comparison method. The comparison references were defined by +2 when one product was markedly superior to the other, and by +1 when relatively good, and expressed by the number of corresponding panelists. The results are shown in Table 4.

**TABLE 4**

| | | Comparative Product | | | Product of the Invention | |
|---|---|---|---|---|---|---|
| | | +2 | +1 | 0 | +1 | +2 |
| 1. | Total evaluation | 0 | 1 | 3 | 5 | 1 |
| 2. | Brushing with fingers when rinsed | 0 | 1 | 3 | 4 | 2 |
| 3. | Flexibility when rinsed | 0 | 2 | 3 | 3 | 2 |
| 4. | Smoothness after drying | 0 | 1 | 3 | 4 | 1 |
| 5. | Tensity and strength after drying | 0 | 2 | 3 | 3 | 2 |
| 6. | Hair entanglement when subsequently shampooing | 0 | 0 | 2 | 5 | 3 |

### EXAMPLE 3

Hair rinses having respective compositions shown in Table 5 (Products of the Invention 10 to 12) were produced in the usual way.

**TABLE 5**

| Components (% by weight) | | Product of the Invention | | |
|---|---|---|---|---|
| | | 10 | 11 | 12 |
| Quaternary ammonium salt | (1-1) | 3.0 | - | - |
| | (1-3) | - | 3.0 | - |
| | (1-5) | - | - | 2.5 |
| Monostearyl trimethylammonium chloride | | - | - | 0.5 |
| Cetanol | | 3.0 | 3.0 | 6.0 |
| Isopropyl palmitate | | 0.5 | 0.5 | - |
| Oleic acid monoglyceride | | - | - | 1.0 |
| Dimethylpolysiloxane (average molecular weight: 9,000) | | 2.0 | 2.0 | 2.0 |
| Polyether modified silicone | | 2.0 | 2.0 | 2.0 |
| 18-Methyleicosanoic acid | | 3.0 | - | - |
| Behenic acid | | - | 3.0 | - |
| Glycerol | | 20.0 | - | 20.0 |
| Propylene glycol | | - | 20.0 | - |
| Pentaerythritol isostearyl glycidyl ether 1 mol addition product | | 1.5 | 1.5 | - |
| Sorbitol isostearyl glycidyl ether 1 mol addition product | | - | - | 1.5 |
| Diethylene glycol monoethyl ether | | 5.0 | - | - |
| Diethylene glycol monobutyl ether | | - | 5.0 | - |
| Benzyl alcohol | | - | - | - |
| Bezyloxyethyl alcohol | | - | - | 2.0 |
| Glycolic acid | | - | - | 4.0 |
| Sodium hydroxide | | - | - | 2.0 |
| Purified water | | balance | balance | balance |

### EXAMPLE 4

Shampoos having respective compositions shown in Table 6 (Products of the Invention 13 to 18) were produced in the usual way.

**TABLE 6**

| Components (% by weight) | Products of the Invention | | | | | |
|---|---|---|---|---|---|---|
| | 13 | 14 | 15 | 16 | 17 | 18 |
| Polyoxyethylene (3) lauryl ether sodium sulfate | 15 | 15 | - | - | - | - |
| Decyl glucoside | - | - | - | - | 15 | - |
| 2-Hydroxy-3-[(2-hydroxyethyl)-[2-(1-oxotetradecyl)aminoethylaminopropyl]-N,N,N-trimethylammonium chloride | - | - | - | - | - | 15 |
| Quaternary ammonium salt (1-2) | 0.5 | - | 0.5 | - | 0.5 | - |
| Quaternary ammonium salt (1-4) | - | 0.5 | - | 0.5 | - | 0.5 |
| Quaternary ammonium salt (1-6) | 0.5 | - | 0.5 | - | 0.5 | - |
| Isostearyl pentaerythryl glycidyl ether 1 mol addition product | 0.5 | 0.5 | 0.5 | - | 0.5 | - |
| Diethylene glycol monobutyl ether | 0.5 | - | - | 0.5 | - | - |
| Behenic acid | 0.5 | - | 0.5 | - | 0.5 | - |
| Glycolic acid | - | 0.5 | - | 0.5 | - | 0.5 |
| Purified water | balance | balance | balance | balance | balance | balance |

These shampoos did not cause entanglement of hair at the time of washing and showed smooth feeling after drying.

### EXAMPLE 5

### (Styling lotion)

A styling lotion having the following composition was produced.

| Component | Amount (% by weight) |
|---|---|
| Quaternary ammonium salt (1-4) | 0.5 |
| Polyethylene glycol | 0.5 |
| Acrylic resin alkanolamine solution | 5.0 |
| 2-Benzyloxyethyl alcohol | 1.0 |
| Methacryl ester polymer | 1.0 |
| Ethanol | 20.0 |
| Perfume | 0.4 |
| Water | balance |
| Total | 100.0 |

### EXAMPLE 6

### (Conditioning foam)

A conditioning foam having the following composition was produced.

| Component | Amount (% by weight) |
|---|---|
| Quaternary ammonium salt (1-5) | 0.5 |
| Octyldodecyl myristate | 1.0 |
| Dipropylene glycol | 1.0 |
| Glycerol | 2.5 |
| Liquid paraffin | 2.5 |
| Polyoxyethylene(20) sorbitan monostearate | 0.2 |
| Glycolic acid | 0.5 |
| Isostearyl pentaerythryl glycidyl ether 1 mol addition product | 0.5 |
| Ethanol | 5.0 |
| Methylparaben | 0.1 |
| Perfume | 0.1 |
| Propellant (LPG) | 10.0 |
| Water | balance |
| Total | 100.0 |

### EXAMPLE 7

### (Hair cream)

A hair cream having the following composition was produced.

| Component | Amount (% by weight) |
|---|---|
| Quaternary ammonium salt (1-6) | 2.0 |
| Cetyl trimethylammonium chloride | 1.0 |
| Polyoxyethylene(20) sorbitan monostearate | 0.5 |
| Cetyl alcohol | 5.0 |
| Isostearyl pentaerythryl glycidyl ether 1 mol addition product | 0.5 |
| Behenic acid | 1.0 |
| Dipropylene glycol | 6.0 |
| Glycerol | 10.0 |
| Liquid paraffin | 3.0 |
| Perfume | 0.4 |
| Water | balance |
| Total | 100.0 |

### EXAMPLE 8

### (Permanent Hair Dye First Compartment)

A permanent hair dye first compartment having the following composition was produced.

| Component | Amount (% by weight) |
|---|---|
| Paraphenylenediamine | 2.0 |
| 2,4-Diaminoanisole | 1.0 |
| Resorcinol | 0.2 |
| Oleic acid | 10.0 |
| Polyoxyethylene(10) oleyl ether | 15.0 |
| Quaternary ammonium salt (1-1) | 1.0 |
| Propylene glycol | 10.0 |
| Liquid ammonia (28%) | 6.0 |
| Water | balance |
| Frost DS (EDTA 2Na) | 0.5 |
| Total | 100.0 |

### EXAMPLE 9

### (Permanent Wave Agent First Compartment)

A permanent wave agent first compartment having the following composition was produced.

| Component | Amount (% by weight) |
|---|---|
| Ammonium thioglycolate | 6.0 |
| 18-Methyleicosanoic acid | 1.0 |
| Liquid ammonia (28%) | 3.0 |
| Frost DS (EDTA 2Na) | 0.5 |
| 2-Benzyloxyethyl alcohol | 10.0 |
| Quaternary ammonium salt (1-2) | 2.0 |
| Water | balance |
| Total | 100.0 |

### EXAMPLE 10

### (Permanent Wave Lotion Second Compartment)

A permanent wave ageng second compartment having the following composition was produced.

| Component | Amount (% by weight) |
|---|---|
| Sodium bromate | 8.0 |
| Quaternary ammonium salt (1-2) | 2.0 |
| Isostearic acid | 0.5 |
| 2-Benzyloxyethyl alcohol | 5.0 |
| Water | balance |
| Total | 100.0 |

## Claims

1. A hair cosmetic comprising a quaternary ammonium salt represented by the following formula (1): 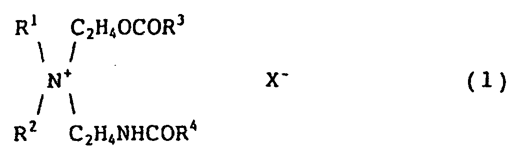 wherein R¹ and R² may be the same or different and each represents an alkyl or hydroxyalkyl group having 1 to 4 carbon atoms, R³ and R⁴ may be the same or different and each represents a straight-chain or branched alkyl or alkenyl group having 7 to 35 carbon atoms and X⁻ represent an anion.

2. The hair cosmetic of claim 1, which further comprises at least one component selected from the group consisting of fats and oils, silicones, solvents, α-hydroxymonocarboxylic acids and liquid crystal forming base materials.

3. The hair cosmetic of claim 2, wherein said solvent is a compound represented by the following formula: 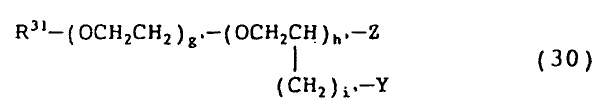 wherein R³¹ represents hydrogen atom, a lower alkyl group or a group represented by 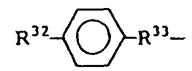 (R³² represents hydrogen atom, methyl group or methoxy group and R³³ represents a bond or a saturated or unsaturated divalent hydrocarbon radical having 1 to 3 carbon atoms), each of Y and Z represents hydrogen atom or hydroxyl group and each of g', h' and i' is an integer of 0 to 5, provided that a case in which g'=h'=i'=0 and Z=H, and a case in which g'=h'=i'=0, R³²=H and Z=OH are excluded.

4. The hair cosmetic of claims 2 and 3, wherein said liquid crystal forming material has at least one long chain branched alkyl or alkenyl group and at least three hydroxyl groups per molecule.

5. The hair cosmetic of anyone of claims 2 to 4, wherein said liquid crystal forming material has at least one long chain branched alkyl or alkenyl group and at least three hydroxyl groups per molecule and gives a lamella crystal structure at a temperature of 25°C and more than 50°C.

6. The hair cosmetic of anyone of claims 2 to 5, wherein said liquid crystal forming material is selected from glycerylated polyols, methyl branched fatty acid esters and branched fatty acid glyceroglycolipids.

## Patentansprüche

1. Haarkosmetisches Produkt, umfassend ein quaternäres Ammoniumsalz, dargestellt durch die folgende Formel (1): 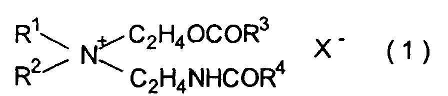 wobei R¹ und R² gleich oder verschieden sein können und jeweils eine Alkyl- oder Hydroxyalkylgruppe mit 1 bis 4 Kohlenstoffatomen darstellt, R³ und R⁴ gleich oder verschieden sein können und jeweils eine geradkettige oder verzweigte Alkyl- oder Alkenylgruppe mit 7 bis 35 Kohlenstoffatomen darstellt, und X ein Anion darstellt.

2. Haarkosmetisches Produkt gemäss Anspruch 1, das weiterhin mindestens eine Komponente, ausgewählt aus der Gruppe, bestehend aus Fetten und Ölen, Siliconen, Lösungsmitteln, α-Hydroxymonocarbonsäuren und Flüssigkristall-bildenden Materialien, umfasst.

3. Haarkosmetisches Produkt gemäss Anspruch 2, wobei das Lösungsmittel eine Verbindung ist, dargestellt durch die folgende Formel: 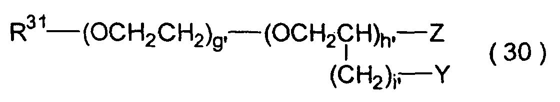 wobei R³¹ ein Wasserstoffatom, eine Niederalkylgruppe oder eine Gruppe, dargestellt durch 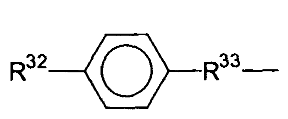 (R³² stellt ein Wasserstoffatom, eine Methylgruppe oder eine Methoxygruppe dar und R³³ stellt eine Bindung oder ein gesättigtes oder ungesättigtes zweiwertiges Kohlenwasserstoffradikal mit 1 bis 3 Kohlenstoffatomen dar) darstellt, sowohl Y wie auch Z ein Wasserstoffatom oder eine Hydroxylgruppe darstellt und g', h' und i' jeweils eine ganze Zahl von 0 bis 5 ist, unter der Massgabe, dass der Fall, in dem g' = h' = i' = 0 und Z = H ist, und der Fall, in dem g' = h' = i' = 0, R³² = H und Z = OH ist, ausgenommen sind.

4. Haarkosmetisches Produkt gemäss den Ansprüchen 2 und 3, wobei das Flüssigkristall-bildende Material mindestens eine langkettige verzweigte Alkyl- oder Alkenylgruppe und mindestens drei Hydroxylgruppen pro Molekül aufweist.

5. Haarkosmetisches Produkt gemäss einem der Ansprüche 2 bis 4, wobei das Flüssigkristall-bildende Material mindestens eine langkettige verzweigte Alkyl- oder Alkenylgruppe und mindestens drei Hydroxylgruppen pro Molekül aufweist und eine Streifenkristallstruktur bei einer Temperatur von 25°C und mehr als 50°C ergibt.

6. Haarkosmetisches Produkt gemäss einem der Ansprüche 2 bis 5, wobei das Flüssigkristall-bildende Material ausgewählt wird aus glycerylierten Polyolen, Methylverzweigten Fettsäureestern und verzweigten Fettsäureglyceroglycolipiden.

## Revendications

1. Cosmétique capillaire comprenant un sel d'ammonium quaternaire représenté par la formule (1) suivante : 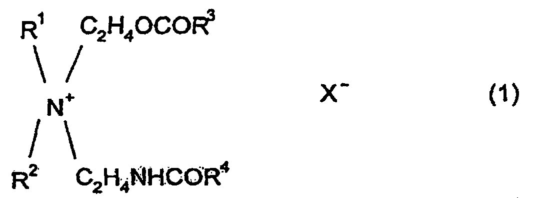 dans laquelle R¹ et R² peuvent être identiques dou différents et chacun représente un groupe alkyle ou hydroxyalkyle ayant de 1 à 4 atomes de carbone, R³ et R⁴ peuvent être identiques ou différents et chacun représente un groupe alkyle ou alcényle à chaîne droite ou ramifiée ayant de 7 à 35 atomes de carbone et X⁻ représente un anion.

2. Cosmétique capillaire selon la revendication 1, comprenant en outre au moins un composant choisi dans le groupe comprenant les graisses et les huiles, les silicones, les solvants, les acides α-hydroxymonocarboxyliques et les substances de base formant des cristaux liquides.

3. Cosmétique capillaire selon la revendication 2, dans lequel ledit solvant est un composé représenté par la formule suivante : 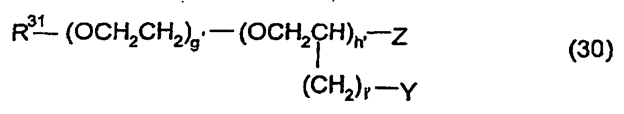 dans laquelle R³¹ représente un atome d'hydrogène, un groupe alkyle inférieur ou un groupe représenté par 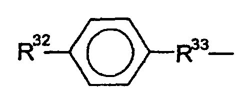 (R³² représente ùn atome d'hydrogène, un groupe méthyle ou un groupe méthoxy et R³³ représente une liaison ou un radical hydrocarboné divalent saturé ou insaturé ayant de 1 à 3 atomes de carbone), chaque élément parmi Y et Z représente un atome d'hydrogène ou un groupe alkyle et chaque élément parmi g', h' et i' est un entier compris entre 0 et 5, à condition d'exclure un cas dans lequel g'=h'=i'=0 et Z=H, et un cas dans lequel g'=h'=i'=0, R³²=H et Z=OH.

4. Cosmétique capillaire selon les revendications 2 et 3, dans lequel ladite substance formant des cristaux liquides possède au moins un groupe alkyle ou alcényle à chaîne longue et ramifiée et au moins trois groupes hydroxyle par molécule.

5. Cosmétique capillaire selon l'une quelconque des revendications 2 à 4, dans lequel ladite substance formant des cristaux liquides possède au moins un groupe alkyle ou alcényle à chaîne longue et ramifiée et au moins trois groupes hydroxyle par molécule et donne une structure cristalline lamellaire à une température comprise entre 25°C et plus de 50°C.

6. Cosmétique capillaire selon l'une quelconque des revendications 2 à 5, dans lequel ladite substance formant des cristaux liquides est choisie parmi les polyols glycérylés, les esters d'acides gras ramifiés de méthyle et les glycéroglycolipides d'acides gras ramifiés.
